# EUROPEAN PATENT APPLICATION

(11) **EP 3 646 875 A1**
(43) Date of publication of application: **06.05.2020**
(21) Application number: 18823384.5
(22) Date of filing: 28.06.2018
(51) Int. Cl.: A61K 31/7004, A61K 9/14, A61K 9/51, A61K 31/44, A61K 45/00, A61K 47/34, A61P 1/16, A61P 35/00, A61P 43/00

(54) **PHARMACEUTICAL COMPOSITION AND TUMOR IMMUNOACTIVITY PROMOTER**

(30) Priority: 28.06.2017 JP 2017125770; 05.10.2017 JP 2017195178
(71) Applicant: Sentan Pharma Inc., Fukuoka-shi, Fukuoka 812-0027 (JP); Kawasaki Gakuen Educational Foundation, Kurashiki-shi, Okayama 701-0192 (JP)
(72) Inventor: HINO Keisuke, Kurashiki-shi Okayama 701-0192 (JP); NISHINA Sohji, Kurashiki-shi Okayama 701-0192 (JP); SASAKI Kyo, Kurashiki-shi Okayama 701-0192 (JP); FUKUDA Kotaro, Uruma-shi Okinawa 904-2234 (JP)
(74) Representative: V.O.
(86) International application number: PCT/JP2018/024528
(87) International publication number: WO 2019/004338

(57) **Abstract**

The present disclosure provides a pharmaceutical composition including biocompatible particles containing a sugar derivative, wherein the biocompatible particles contain a lactic acid-glycolic acid copolymer. The pharmaceutical composition may be used in combination with an anticancer drug.

## Description

### Technical Field

The present disclosure relates to pharmaceutical compositions and an anticancer immune activator.

### Background Art

2-Deoxy-D-glucose (hereinafter, simply referred to as "2DG"), which is a derivative of glucose, is taken up by cells by a glucose transporter, in the same manner as glucose. 2DG which has been taken up by and accumulated in cancer cells has an anticancer effect, which works through the inhibition of the production of ATP (adenosine triphosphate) associated with the inhibition of the glycolytic pathway.

In the treatment of cancer, the combined use of 2DG with an anticancer drug whose mechanism of action is different from that of 2DG has been investigated. For example, Patent Literature 1 discloses that the administration of 2DG and paclitaxel to mice transplanted with a human non-small-cell lung cancer cell line results in an increased anticancer effect, as compared to the administration of paclitaxel alone.

Further, Non Patent Literature 1 discloses the results of a dose-escalation trial of 2DG combined with docetaxel by oral administration, as a phase 1 clinical trial.

### Citation List

### Patent Literature

PTL 1: Unexamined Japanese Patent Application Kokai Publication (Translation of PCT Application) No. 2006-515883

### Non Patent Literature

NPL 1: Luis E. Raez, and 14 others, "A phase I dose-escalation trial of 2-deoxy-D-glucose alone or combined with docetaxel in patients with advanced solid tumors.", Cancer Chemotherapy and Pharmacology, 2013, 71, p. 523 to 530

### Summary of Invention

### Technical Problem

According to Non Patent Literature 1, 2DG was tolerated up to a dose of 63 mg/kg. However, severe side-effects such as gastrointestinal bleeding, long QT syndrome and hyperglycemia were observed at a dose of from 63 to 88 mg/kg. For the clinical application of 2DG, the reduction of side-effects, an improvement in anticancer effect and the like are required.

The present disclosure has been made in view of the above described situation, and an objective of the present disclosure is to provide pharmaceutical compositions and an anticancer immune activator, which enable to achieve a high anticancer effect with less side-effects.

### Solution to Problem

A pharmaceutical composition according to Aspect 1 of the present disclosure includes biocompatible particles containing a sugar derivative,
wherein the biocompatible particles contain a lactic acid-glycolic acid copolymer.

In this case, the pharmaceutical composition according to Aspect 1 of the present disclosure may be used in combination with an anticancer drug.

A pharmaceutical composition according to Aspect 2 of the present disclosure includes an anticancer drug, and is used in combination with the pharmaceutical composition according to Aspect 1 of the present disclosure.

A pharmaceutical composition according to Aspect 3 of the present disclosure includes:
biocompatible particles containing a sugar derivative; and
an anticancer drug;
wherein the biocompatible particles contain a lactic acid-glycolic acid copolymer.

The above described anticancer drug may be sorafenib, doxorubicin or cis-platin.

The above described sugar derivative may be 2-deoxy-D-glucose.

The above described biocompatible particles may have an average particle size of from 50 to 170 nm.

Further, the pharmaceutical composition according to any one of Aspect 1, Aspect 2 and Aspect 3 of the present disclosure may be used for treating hepatocellular carcinoma, renal cancer, colorectal cancer or pancreatic cancer.

An anticancer immune activator according to Aspect 4 of the present disclosure includes biocompatible particles containing a sugar derivative, wherein the biocompatible particles contain a lactic acid-glycolic acid copolymer.

### Advantageous Effects of Invention

According to the present disclosure, a high anticancer effect can be obtained with less side-effects.

### Brief Description of Drawings

FIG. 1 shows the appearance of nude mice subcutaneously transplanted with a hepatoma cell line, according to Example 1, and the appearance of tumors removed from the mice;
FIG. 2 is a graph showing changes over time in the tumor volume in the nude mice subcutaneously transplanted with the hepatoma cell line, according to Example 1;
FIG. 3 is a graph showing changes over time in the body weight of the nude mice subcutaneously transplanted with the hepatoma cell line, according to Example 1;
FIG. 4 is a graph showing changes over time in the amount of meals taken by the nude mice subcutaneously transplanted with the hepatoma cell line, according to Example 1;
FIG. 5 is a graph showing changes over time in the blood glucose level after the administration of a 2DG solution, in the high dose 2DG group according to Example 1;
FIG. 6 is a graph showing the particle size distribution of 2DG nanoparticles according to Example 2;
FIG. 7 shows the appearance of nude mice subcutaneously transplanted with the hepatoma cell line, according to Example 3, and the appearance of tumors removed from the mice;
FIG. 8 is a graph showing changes over time in the tumor volume in the nude mice subcutaneously transplanted with the hepatoma cell line, according to Example 3;
FIG. 9 is a graph showing changes over time in the body weight of the nude mice subcutaneously transplanted with the hepatoma cell line, according to Example 3;
FIG. 10 is a graph showing changes over time in the amount of meals taken by the nude mice subcutaneously transplanted with the hepatoma cell line, according to Example 3;
FIG. 11 is a graph showing changes over time in the blood glucose level of the mice according to Example 3, after the administration of the 2DG nanoparticles;
FIG. 12 is a graph (No. 1) showing changes over time in the tumor volume in nude mice subcutaneously transplanted with the hepatoma cell line, according to Example 4;
FIG. 13 shows the appearance (No. 1) of the nude mice subcutaneously transplanted with the hepatoma cell line, according to Example 4, and the appearance of tumors removed from the mice;
FIG. 14 is a graph (No. 2) showing changes over time in the tumor volume in the nude mice subcutaneously transplanted with the hepatoma cell line, according to Example 4;
FIG. 15 shows the appearance (No. 2) of the nude mice subcutaneously transplanted with the hepatoma cell line, according to Example 4, and the appearance of tumors removed from the mice;
FIG. 16 is a graph showing changes over time in the tumor volume in nude mice subcutaneously transplanted with a renal cancer cell line, according to Example 5;
FIG. 17 shows the appearance of the nude mice subcutaneously transplanted with the renal cancer cell line, according to Example 5, and the appearance of tumors removed from the mice;
FIG. 18 is a graph showing changes over time in the tumor volume in nude mice subcutaneously transplanted with a colorectal cancer cell line, according to Example 6;
FIG. 19 shows the appearance of the nude mice subcutaneously transplanted with the colorectal cancer cell line, according to Example 6, and the appearance of tumors removed from the mice;
FIG. 20 is a graph showing changes over time in the tumor volume in nude mice subcutaneously transplanted with a pancreatic cancer cell line, according to Example 7;
FIG. 21 shows the appearance of the nude mice subcutaneously transplanted with the pancreatic cancer cell line, according to Example 7, and the appearance of tumors removed from the mice;
FIG. 22 shows changes over time in the biodistribution of a pigment in the tumors and the body of a nude mouse subcutaneously transplanted with the hepatoma cell line, according to Example 8;
FIG. 23 shows the distribution of the pigment in tumor tissue and respective organs of the nude mouse subcutaneously transplanted with the hepatoma cell line, shown in FIG. 22, 7 days after the administration of pigment-containing nanoparticles;
FIG. 24 is a graph showing changes over time in the tumor volume in nude mice subcutaneously transplanted with the hepatoma cell line, according to Example 9;
FIG. 25 shows the appearance of the nude mice subcutaneously transplanted with the hepatoma cell line, according to Example 9, and the appearance of tumors removed from the mice;
FIG. 26 shows the appearance of hepatocarcinogenic mice according to Example 10;
FIG. 27 is a graph showing the maximum diameter of the tumors in the hepatocarcinogenic mice according to Example 10;
FIG. 28 is a graph showing the total tumor volume in the hepatocarcinogenic mice according to Example 10; and
FIG. 29 shows the results of immunostaining of the liver tumor tissue of the hepatocarcinogenic mice according to Example 10.

### Description of Embodiments

### (Embodiments)

The embodiments according to the present disclosure will now be described. A pharmaceutical composition according to the present embodiment includes biocompatible particles containing a sugar derivative. For example, the sugar derivative is a derivative of D-glucose, which inhibits the glycolytic pathway.

The sugar derivative is preferably a glucose derivative which does not have a hydroxyl group at the position 2 of the glucose ring. Examples of the glucose derivative include mannose, galactose and 5-thio-glucose. The glucose derivative may have a fluorine atom instead of a hydrogen atom at an arbitrary position on the glucose ring. Examples of the glucose derivative having a fluorine atom include 2-fluoro-2-deoxy-D-glucose and 2-difluoro-2-D-deoxy-glucose. The glucose derivative may have an amino group instead of a hydroxyl group at an arbitrary position other than position 6 on the glucose ring. Examples of the glucose derivative having an amino group include 2-amino-2-deoxy-D-glucose and 2-amino-2-deoxy-D-galactose. Examples of the glucose derivative include, in addition to those mentioned above, 2-F-mannose, 2-mannosamine, 2-deoxygalactose and 2-F-deoxygalactose. The sugar derivative is suitably 2DG.

In the pharmaceutical composition according to the present embodiment, the biocompatible particles contain the above described sugar derivative. The biocompatible particles are particles which cause less irritation or are less toxic to a living body, and which have characteristics to be decomposed and metabolized after administration. The term "to contain" as used herein means that the sugar derivative may be enclosed or encapsulated within the biocompatible particles, or that the sugar derivative is enclosed or encapsulated within the biocompatible particles and a part of the sugar derivative may be exposed on the surfaces of the biocompatible particles.

The biocompatible particles contain a lactic acid-glycolic acid copolymer (also referred to as a "polylactide glycolide copolymer"; hereinafter, simply referred to as "PLGA"). PLGA is a copolymer comprising lactic acid or lactide and glycolic acid or glycolide, for example, in a ratio of from 1:99 to 99:1, preferably in a ratio of 75:25, and more preferably in a ratio of 3:1. PLGA may be synthesized from arbitrarily selected monomers by a known method, or a commercially available PLGA may be used. The commercially available PLGA may be, for example, PLGA 7520 (the ratio of lactic acid and glycolic acid = 75:25, weight-average molecular weight: 20,000, manufactured by Wako Pure Chemical Industries, Ltd.). A PLGA in which the contents of lactic acid and glycolic acid are from 25% by weight to 65% by weight is preferred, because such a PLGA is an amorphous copolymer, and is soluble in an organic solvent, such as acetone.

The biocompatible particles have a particle size of less than 1,000 nm, for example, from 2.5 to 900 nm, preferably from 5 to 500 nm or from 10 to 300 nm, more preferably from 30 to 250 nm, still more preferably from 40 to 180 nm, and particularly preferably from 50 to 100 nm. For example, the biocompatible particles have a particle size of from 50 to 170 nm. The particle size of the biocompatible particles can be measured, for example, by a method such as sieving, sedimentation, microscopy, light scattering, laser diffraction/scattering, electrical resistance test, observation using a transmission electron microscope, observation using a scanning electron microscope, or the like. The particle size may also be measured using a particle size distribution analyzer. The particle size of the biocompatible particles can be represented by the Stokes equivalent diameter, the circle equivalent diameter or the sphere equivalent diameter, depending on the measurement method. Further, the particle size may be the average particle size, the volume average particle size, the area average particle size or the like, which is determined by measuring a plurality of particles and calculating the average value thereof. The particle size may also be the average particle size calculated from the number distribution or the like based on the measurement by the laser diffraction/scattering method or the like.

Specifically, the particle size may also be 50% diameter (D₅₀). The 50% diameter (D₅₀) refers, when a cumulative curve is drawn taking the total volume of the population of particles as 100%, to the particle size at the point where the cumulative curve is 50%. The cumulative curve and the D₅₀ can be determined using a commercially available particle size distribution analyzer. The particle size distribution analyzer may be, for example, NIKKISO Nanotrac Wave-EX150 (manufactured by Nikkiso Co., Ltd.).

The particle size distribution of the biocompatible particles preferably has a single peak, in order to enhance the extent of bioavailability of the sugar derivative contained in the biocompatible particles. The span value, which is an index indicating the uniformity of the particle size of the biocompatible particles, is determined by: (D₉₀-D₁₀)/ D₅₀. The D₉₀ as used herein refers to 90% diameter, which is the particle size at the point where the above described cumulative curve is 90%. The D₁₀ as used herein refers to 10% diameter, which is the particle size at the point where the above described cumulative curve is 10%. The D₉₀ and D₁₀ can be determined using a commercially available particle size distribution analyzer. The span value is not limited as long as the effect of the biocompatible particles can be obtained. However, the span value is, for example, 2.5 or less, preferably 2.3 or less, more preferably 2.0 or less or 1.8 or less, and particularly preferably 1.6 or less.

The surfaces of the biocompatible particles may be modified with polyethylene glycol (PEG). For example, it is possible to obtain biocompatible particles whose surfaces are modified with PEG, by using a PEG-modified product of PLGA in the production of the biocompatible particles. When the surfaces of the biocompatible particles are modified with PEG, stability of the biocompatible particles in blood will be improved.

The biocompatible particles containing a sugar derivative can be produced using an arbitrary known production method. For example, the biocompatible particles can be produced by emulsification in water. In the emulsification in water, two types of solvents, namely, a good solvent in which PLGA dissolves and a poor solvent in which PLGA does not dissolve are used. As the good solvent, an organic solvent in which PLGA dissolves and which can be mixed with a poor solvent is used. The types of the good solvent and the poor solvent are not particularly limited.

Examples of the poor solvent include water. In cases where water is used as the poor solvent, a surfactant may be added to the water. For example, the surfactant is preferably polyvinyl alcohol (PVA). Examples of the surfactant other than PVA include lecithin, hydroxymethyl cellulose and hydroxypropyl cellulose.

Examples of the good solvent include: halogenated alkanes which are organic solvents that have a low boiling point and that are poorly soluble in water; acetone; methanol; ethanol; ethyl acetate; diethyl ether; cyclohexane; benzene; and toluene. For example, acetone alone, or a mixed liquid of acetone and ethanol is preferably used, because of having less adverse effects on the environment and the human body.

In the emulsification in water, PLGA is dissolved in a good solvent, first, and then a solution of the sugar derivative is added to and mixed with the good solvent so as to prevent the precipitation of PLGA. When the resulting mixed liquid containing PLGA and the sugar derivative is added dropwise to a poor solvent while stirring, the good solvent in the mixed liquid quickly diffuses into the poor solvent. As a result, the emulsification of the good solvent occurs in the poor solvent, to form emulsion droplets of the good solvent.

Further, the mutual diffusion of the good solvent and the poor solvent causes the organic solvent to continuously diffuse into the poor solvent from within the emulsion, resulting in a decrease in the solubility of the PLGA and the sugar derivative within the emulsion droplets. Finally, the biocompatible particles as spherical crystal particles containing the sugar derivative are produced. Thereafter, the organic solvent as the good solvent is removed by centrifugation or distillation under reduced pressure, to obtain a powder of biocompatible particles. The resulting powder is used as it is, or subjected to freeze drying or the like, as necessary, to be compounded into agglomerated particles which can be redispersed. The content percentage of the sugar derivative in the resulting biocompatible particles is, for example, from 0.01 to 99% by weight, from 0.1 to 30% by weight, from 0.5 to 20% by weight, from 1 to 15% by weight, from 3 to 10% by weight, or from 5 to 10% by weight. The content percentage of the sugar derivative as used herein refers to the percentage of the weight of the sugar derivative with respect to the weight of the biocompatible particles. The content percentage of the sugar derivative can be determined by quantifying the weight of the sugar derivative extracted from a predetermined weight of the nanoparticles, and calculating the percentage of the weight of the sugar derivative with respect to the weight of the biocompatible particles.

It is also possible to add a cationic polymer to the poor solvent, in order to increase the content percentage of the sugar derivative in the biocompatible particles. Examples of the cationic polymer include: chitosan and chitosan derivatives; cationized cellulose obtained by allowing a plurality of cation groups to bind to cellulose; and polyamino compounds such as polyethyleneimine, polyvinylamine and polyallylamine.

The biocompatible particles containing a sugar derivative are suitably produced using a forced thin-film microreactor. In the case of using the forced thin-film microreactor, the above described good solvent and poor solvent are first introduced between processing surfaces, which are provided so as to face each other, and at least one of which surfaces is rotatable relative to the other. In the thin film fluid thereby formed, the good solvent and the poor solvent are mixed, so as to allow the biocompatible particles containing the sugar derivative to precipitate in the thin film fluid. As the forced thin-film microreactor, ULREA SS-11 (manufactured by M Technique Co., Ltd.) is preferably used.

The pharmaceutical use of the pharmaceutical composition according to the present embodiment is not particularly limited. This pharmaceutical composition is preferably used as an anticancer drug in the treatment of cancer, particularly in the treatment of solid cancer. Specific examples of the solid cancer include hepatocellular carcinoma, stomach cancer, pancreatic cancer, lung cancer, colorectal cancer, breast cancer, hepatic cancer (liver cancer), renal cancer (kidney cancer), tongue cancer, thyroid cancer, uterine cancer, ovarian cancer, prostate cancer, osteosarcoma, chondrosarcoma, rhabdomyosarcoma and leiomyoma. This pharmaceutical composition is suitably used in the treatment of cancer in which a hypervascular tumor is formed. The hypervascular tumor refers to a tumor in which, when examined by contrast radiography such as contrast computed tomography, abundant arterial blood flow enhanced by contrast radiography is observed. The type of cancer in which a hypervascular tumor is formed is, for example, hepatocellular carcinoma and renal cell carcinoma.

The above described pharmaceutical composition is useful as an anti-hepatocellular carcinoma drug, an anti-renal cancer drug, an anti-colorectal cancer drug, or an anti-pancreatic cancer drug. In another embodiment, the pharmaceutical composition is used as an anticancer drug.

The pharmaceutical composition increases the infiltration of T cells into the interior of a tumor, as will be shown in the following Example 10. Therefore, in still another embodiment, the pharmaceutical composition is used as an anticancer immune activator.

The route of administration of the pharmaceutical composition according to the present embodiment to humans is not particularly limited. The pharmaceutical composition is preferably used as an injectable solution or a drug for oral administration. In this case, the pharmaceutical composition may be, for example, a mixture with a pharmaceutically acceptable carrier. The term "pharmaceutically acceptable carrier" refers to any of various types of organic carrier substances and inorganic carrier substances which are used as materials for pharmaceutical preparations. The pharmaceutically acceptable carrier is incorporated into the pharmaceutical composition, for example, as a vehicle, a lubricant, a binder or a disintegrating agent in a solid pharmaceutical preparation, or as a solvent, a solubilizer, a suspending agent, a tonicity agent, a buffer or a soothing agent in a liquid pharmaceutical preparation, or the like. Further, any of additives such as an antiseptic, an antioxidant, a colorant and a sweetener can be used as necessary.

The pharmaceutical composition according to the present embodiment is produced using a known method, and includes, as an effective ingredient, from 0.000001 to 99.9% by weight, from 0.00001 to 99.8% by weight, from 0.0001 to 99.7% by weight, from 0.001 to 99.6% by weight, from 0.01 to 99.5% by weight, from 0.1 to 99% by weight, from 0.5 to 60% by weight, from 1 to 50% by weight or from 1 to 20% by weight of the biocompatible particles.

The dose of the pharmaceutical composition according to the present embodiment is determined as appropriate depending on the gender, age, body weight, symptoms and the like of a patient. This pharmaceutical composition is administered in such an amount that an effective amount of the sugar derivative is contained therein. The "effective amount" as used above refers to the amount of the sugar derivative required for obtaining the desired result, and is the amount thereof necessary to delay, inhibit, prevent or reverse the progress of the condition, or to cure the condition, to be treated.

The dose of this pharmaceutical composition is typically from 0.01 mg/kg to 1,000 mg/kg, preferably from 0.1 mg/kg to 200 mg/kg, and more preferably from 0.2 mg/kg to 20 mg/kg, and the pharmaceutical composition can be administered once a day, or more than once a day in divided doses. In cases where the pharmaceutical composition is administered in divided doses, the pharmaceutical composition is preferably administered 1 to 4 times a day. Further, the pharmaceutical composition may be administered in any of various types of dosing frequencies, such as, for example, every day, every other day, once a week, every other week, and once a month. Preferably, the dosing frequency is easily determined by a medical doctor and the like. If necessary, a dose other than the above described range can also be used.

The pharmaceutical composition according to the present embodiment may be used in combination with an anticancer drug. To be "used in combination" means that the pharmaceutical composition and an anticancer drug are administered to the same patient in a predetermined period. In the combined use of the pharmaceutical composition with an anticancer drug, the pharmaceutical composition and the anticancer drug are preferably administered simultaneously in time, but may be administered separately at different time points, such as, for example, administering one while the effect of the other is still present. In the case of the combined use, the route of administration of the pharmaceutical composition and that of the anticancer drug may be the same as, or different from, each other. For example, in the case of the combined use, the pharmaceutical composition and the anticancer drug are administered for a predetermined period, in accordance with one regimen in which the dose and usage of each of the pharmaceutical composition and the anticancer drug are defined.

The anticancer drug to be used in combination with the pharmaceutical composition is not particularly limited, and any anticancer drug which is effective for cancer, for example, an anticancer drug having an anticancer effect can be used. Examples of the anticancer drug include alkylating agents, anti-metabolites, anticancer antibiotics, plant alkaloids, platinum pharmaceutical preparations (platinum compound pharmaceutical preparations), biological therapeutics, hormonal agents and molecular target drugs. Specific examples of the anticancer drug include cis-platin, carboplatin, oxaliplatin, cyclophosphamide, methotrexate, fluorouracil, doxorubicin, irinotecan, gemcitabine hydrochloride, tegafur-uracil and docetaxel. The pharmaceutical composition is suitably used for the treatment of hepatocellular carcinoma, in combination with sorafenib, which is a molecular target drug, as an anticancer drug. Further, the pharmaceutical composition may be used in combination with doxorubicin or cis-platin.

As described above in detail, the sugar derivative is contained in the biocompatible particles containing PLGA, in the pharmaceutical composition according to the present embodiment. Therefore, it is possible to selectively and efficiently accumulate the sugar derivative in the target cancer tissue, by the enhanced permeability and retention (EPR) effect. This makes it possible to minimize the impact on normal tissues, thereby reducing side-effects. Further, a high anticancer effect can be obtained by transferring the sugar derivative to the target cancer tissue at a high efficiency.

In the present embodiment, the pharmaceutical composition may be used in combination with an anticancer drug. The sugar derivative contained in the pharmaceutical composition is taken up by cancer cells and inhibits the glycolytic pathway, and as a result, the production of energy and the synthesis of substances due to glucose metabolism are inhibited, thereby exhibiting an anticancer effect. By using an anticancer drug having another mechanism of action, such as, for example, sorafenib, which is a kinase inhibitor, in combination with the sugar derivative, a further anticancer effect can be obtained.

In another embodiment, a pharmaceutical composition including the above described anticancer drug is provided. This pharmaceutical composition is used in combination with the above described pharmaceutical composition including the biocompatible particles containing the sugar derivative. The content of the anticancer drug contained in this pharmaceutical composition as an effective ingredient is the same as the content of the biocompatible particles in the above described pharmaceutical composition. Further, the dose and the administration method of this pharmaceutical composition are also the same as those of the above described pharmaceutical composition including the biocompatible particles.

A pharmaceutical composition according to another embodiment includes the biocompatible particles containing the sugar derivative, and the anticancer drug. In other words, this pharmaceutical composition contains both components, namely, the biocompatible particles containing the sugar derivative and the anticancer drug, within a single pharmaceutical preparation. This makes it possible to administer both the sugar derivative and the anticancer drug simultaneously and by the same route of administration. The weight ratio of the biocompatible particles and the anticancer drug in this pharmaceutical composition is not particularly limited, and adjusted as appropriate depending on the anticancer effect and the like. The weight ratio of the biocompatible particles and the anticancer drug in this pharmaceutical composition is preferably adjusted depending on the weight ratio of the sugar derivative contained in the biocompatible particles and the anticancer drug.

In another embodiment, a method of treating or preventing cancer in a patient by administering to the patient the biocompatible particles containing the sugar derivative, is provided. In this case, the anticancer drug may further be administered to the patient.

### Examples

The present disclosure will now be described further specifically with reference to the following Examples. However, the present disclosure is in no way limited to the following Examples.

### (Example 1: Examination of Anticancer Effect of 2DG, Using Nude Mice Subcutaneously Transplanted with Hepatoma Cell Line)

The Hepatoma cell line Huh-7 (obtained from Cell Resource Center for Biomedical Research, Cell Bank, Institute of Development, Aging and Cancer, National University Corporation Tohoku University) to be transplanted to the mice was cultured at 37°C using a 5% CO₂ incubator. Dulbecco's modified Eagle's medium (DMEM) containing 10% fetal bovine serum was used as the medium for culturing Huh-7 cells.

Six-week-old nude mice (male, BALBc-nu/nu mice) were subcutaneously transplanted with 1 × 10⁷ Huh-7 cells. In order to confirm the survival and growth of the transplanted cells, the measurement of tumor size was carried out using a pair of vernier calipers. At the time point when the tumors in the mice had reached a volume of from 100 to 150 mm³, the mice were divided into the following six groups.
(a) Control group: 200 µL of PBS was administered intraperitoneally every day.
(b) Low dose 2DG group: 100 mg/kg/day of a 100 mg/mL 2DG solution, prepared by dissolving 2DG in PBS, was administered intraperitoneally every day.
(c) High dose 2DG group: 1,000 mg/kg/day of the 100 mg/mL 2DG solution was administered intraperitoneally every day.
(d) Sorafenib group: 5 mg/kg/day of a solution of sorafenib (manufactured by Santa Cruz Biotechnology, Inc.) was administered orally every day, using a sonde, and 200 µL of PBS was administered intraperitoneally every day. The sorafenib solution was prepared by dissolving the bulk powder of sorafenib in 100% DMSO and, then further diluting the solution to 1/20 with olive oil (finally obtained as a 5% DMSO solution).
(e) Sorafenib + low dose 2DG group: 5 mg/kg/day of the sorafenib solution was administered orally every day, using a sonde, and 100 mg/kg/day of the 2DG solution was administered intraperitoneally every day.
(f) Sorafenib + high dose 2DG group: 5 mg/kg/ day of the sorafenib solution was administered orally every day, using a sonde, and 1,000 mg/kg/day of the 2DG solution was administered intraperitoneally every day.

In each of the groups, the tumor volume and the body weight of the mice were measured every 3 days from the start of administration. At the time points of 7 days, 14 days and 21 days after the start of administration, the average amount of meals taken per day was calculated from the amount of meals taken in the previous week. On day 14 after the start of administration, blood was collected from the mice in the high dose 2DG group at a predetermined time, and the blood glucose level was measured using Glucocard (trademark) MyDia (manufactured by Arkray, Inc.). Further, 21 days after the start of administration, the tumors were observed, blood was collected from the mice in accordance with a conventional method, and the livers were removed from the mice. The serological profiles of the collected blood samples were measured using SPOTCHEM (trademark) EZ SP-4430 system (manufactured by Arkray, Inc.). In addition, the weight of the removed livers was measured.

### (Results)

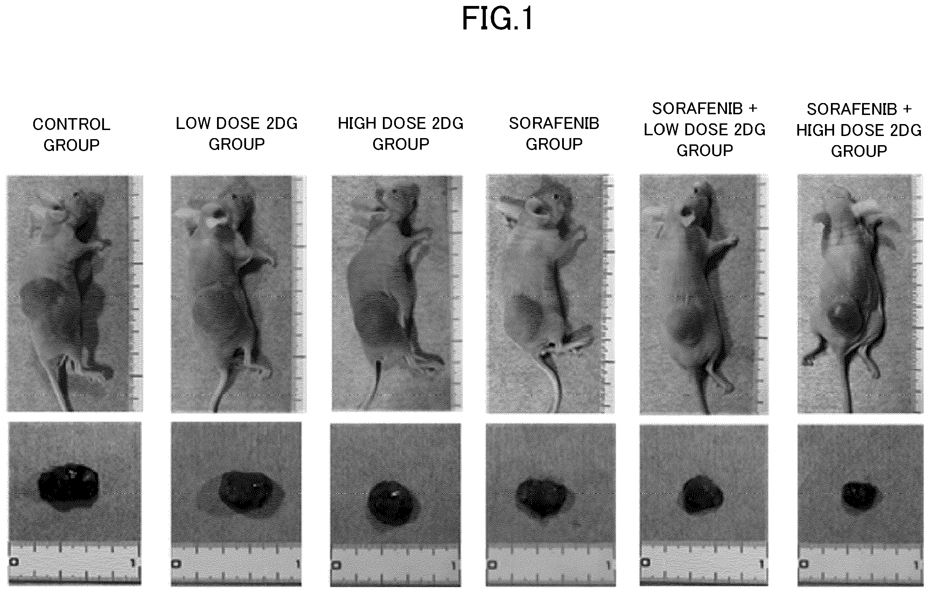

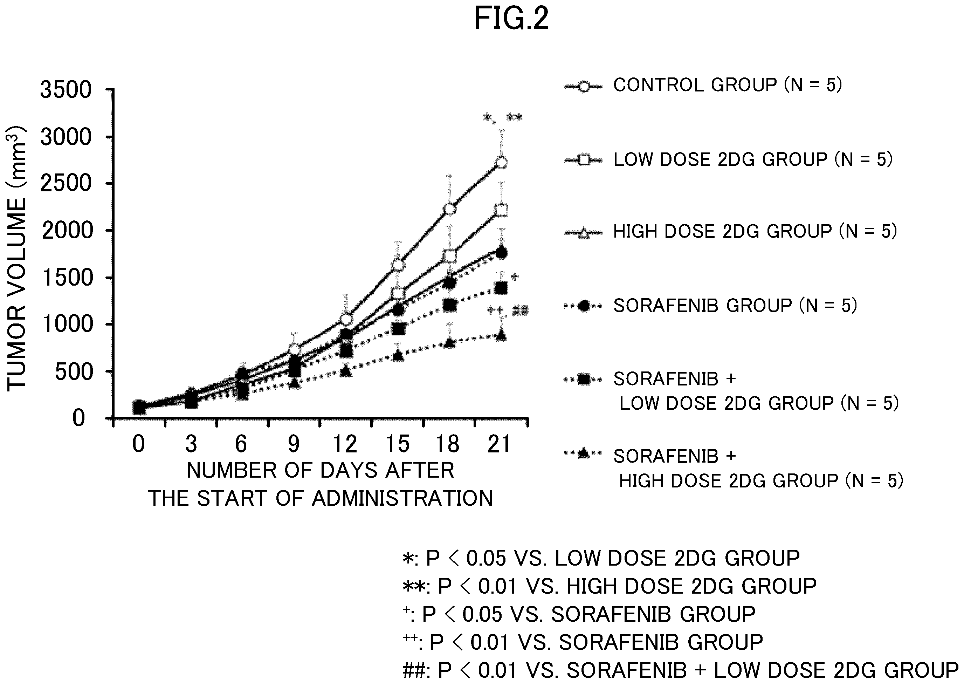

FIG. 1 shows the appearance of the mice 21 days after the start of administration, in the respective groups, and the appearance of the tumors removed from the mice. 2DG decreased the size of the tumors derived from the transplanted hepatoma cells. 2DG further decreased the tumor size, when used in combination with sorafenib. FIG. 2 is a graph showing changes over time in the tumor volume. A significant decrease in the tumor volume in a dose-dependent manner was observed in the low dose 2DG group and the high dose 2DG group. In the sorafenib + low dose 2DG group and the sorafenib + high dose 2DG group, a further significant decrease in the tumor volume in a dose-dependent manner was observed as a result of the combined use of 2DG with sorafenib.

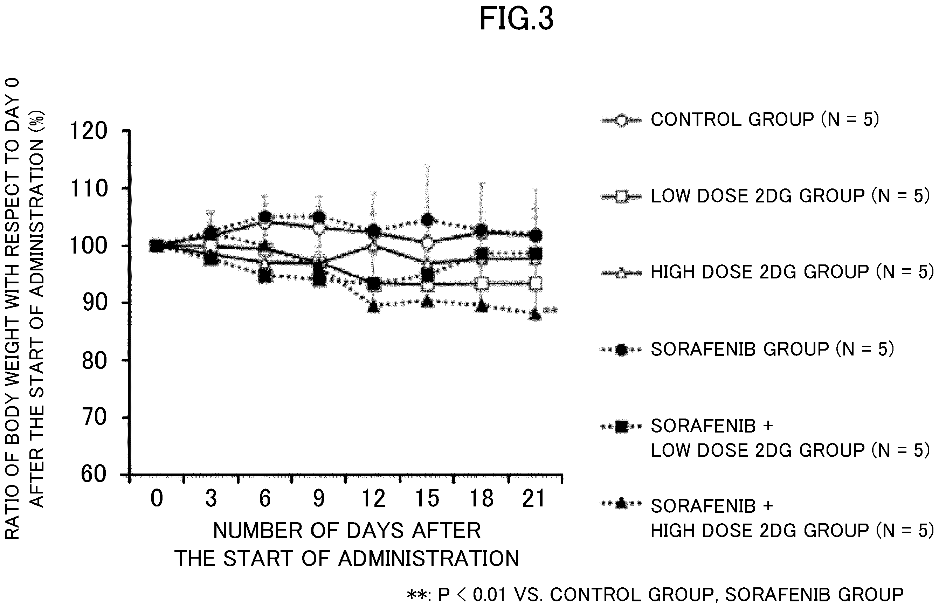

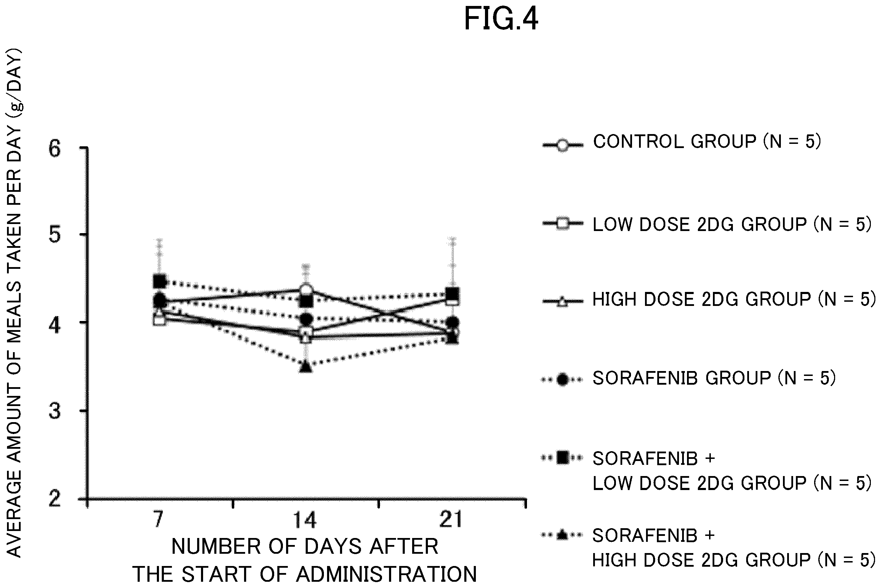

As shown in FIG. 3, the body weight of the mice in the sorafenib + high dose 2DG group was significantly decreased during the period of the experiment, as compared to the control group and the sorafenib group. FIG. 4 shows the average amount of meals taken per day, at the time points of 7 days, 14 days and 21 days after the start of administration. No significant difference was observed in the average amount of meals taken per day, between all the groups. The serological profiles and the liver weight at the time point of 21 days after the start of administration are shown in Table 1. A significantly high ALT value was observed in the sorafenib + high dose 2DG group. Further, a significantly low value of neutral fat was observed in the high dose 2DG group and in the sorafenib + high dose 2DG group.

**[Table 1]**

| | Control group | Low dose 2DG group | High dose 2DG group | Sorafenib group | Sorafenib + low dose 2DG group | Sorafenib + High dose 2DG group |
|---|---|---|---|---|---|---|
| ALT (mg/dL) | 32.4 ± 14.4^{**} | 34.8 ± 16.2^{**} | 69.6 ± 48.2^{*} | 35.0 ± 10.3^{**} | 34.8 ± 13.7^{**} | 122.8 ± 33.7 |
| Liver weight (g) | 1.23 ± 0.1^{*} | 1.21 ± 0.1^{*} | 1.10 ± 0.1 | 1.19 ± 0.1 | 1.22 ± 0.3^{*} | 0.92 ± 0.1 |
| Fasting blood glucose (mg/dL) | 92.4 ± 18.2 | 79.0 ± 10.9 | 77.2 ± 28.0 | 84.8 ± 11.8 | 80.6 ± 17.2 | 58.8 ± 22.0 |
| Total cholesterol (mg/dL) | 121.0 ± 10.7 | 122.6 ± 19.8 | 110.8 ± 23.6 | 120.4 ± 17.8 | 122.2 ± 12.9 | 106.2 ± 37.5 |
| Neutral fat (mg/dL) | 84.8 ± 32.8^{*} | 103.2 ± 24.7^{**,#} | 35.4 ± 18.4 | 95.2 ± 35.7^{**,##} | 97.0 ± 35.8^{**,##} | 27.4 ± 5.4 |

| | | | | | | |
|---|---|---|---|---|---|---|
| ^{**:} P < 0.001, *: P < 0.05 vs. Sorafenib + high dose 2DG group ^{##}: P < 0.001, ^{##}: P < 0.05 vs. high dose 2DG group | | | | | | |

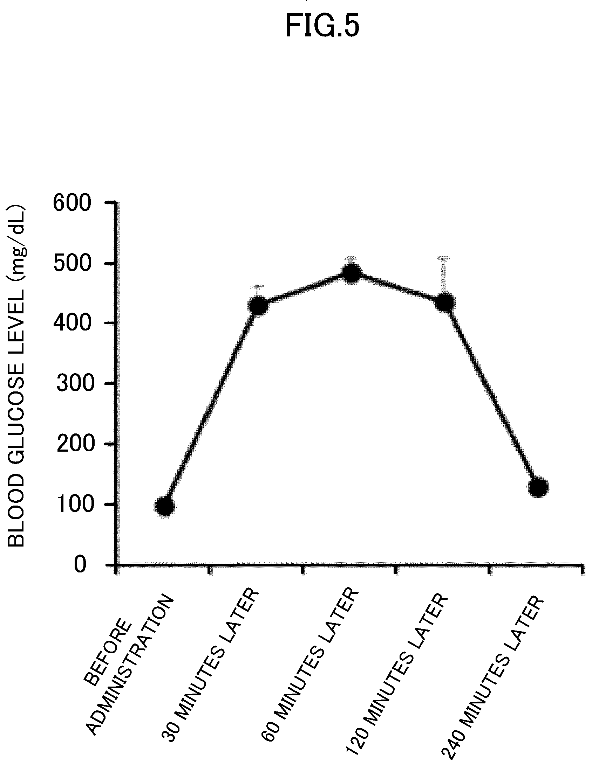

FIG. 5 shows changes over time in the blood glucose level in the high dose 2DG group, after the administration of the 2DG solution, on day 14 after the start of administration. In the mice of the high dose 2DG group, the disturbance of consciousness was observed, along with a marked increase in the blood glucose level. The above results have shown that the administration of a high dose of 2DG could cause a decrease in body weight, liver damage and hyperglycemia, as side effects.

### (Example 2: Preparation of 2DGNanoparticles and Analysis of 2DG Nanoparticles)

Using ULREA SS-11 (manufactured by M Technique Co., Ltd.), 2DG nanoparticles (2DG-PLGA) were prepared as follows. First, Liquid A was filled in a Liquid A tank, and the tank was pressurized to 0.3 MPa. Subsequently, the Liquid A was transferred at a set value of 43°C and at a rate of 167 mL/ min, and thereafter, Liquid B was transferred at a set value of 41°C (measured value: about 29°C) and at a rate of 100 mL/min. The Liquid A is an aqueous solution containing 0.5% PVA. The Liquid B is a solution containing PLGA, 2DG, acetone and ethanol in a weight ratio of 0.65:0.25:66:33. The number of revolutions was adjusted to 1,000 rpm and the back pressure was adjusted to 0.02 MPa. A quantity of 400.5 mL of the discharge liquid was collected, and the removal of the solvents contained in the discharge liquid was carried out for 80 minutes by distillation using an evaporator. As the PLGA to be contained in the Liquid B, PLGA-7520 (lactic acid:glycolic acid = 75:25, weight-average molecular weight: 20,000; manufactured by Wako Pure Chemical Industries, Ltd.) was used. A quantity of 224 mL of the resulting aqueous 2DG-PLGA dispersion was freeze dried, to obtain 2.16 g of 2DG-PLGA.

A quantity of 3.7 mg of 2DG-PLGA prepared as described above was weighed into a tube, and 3.7 mL of Milli-Q water was added thereto. The resulting mixture was stirred using a vortex mixer for 30 seconds, and then subjected to an ultrasonic treatment for 5 minutes. Using Milli-Q water as a control, the particle size distribution of 2DG-PLGA was measured using NIKKISO Nanotrac Wave-EX1 50 (manufactured by Nikkiso Co., Ltd.).

The content percentage of 2DG in 2DG-PLGA was evaluated as follows. First, standard solutions were prepared. A quantity of 200 mg of 2DG was weighed into a 100 mL measuring flask, and 50 mL of water was added to dissolve 2DG. Next, the resulting solution was diluted with acetonitrile in the measuring flask to a total volume of 100 mL, to be used as a standard stock solution (2,000 µg/mL). The thus prepared stock solution was serially diluted to concentrations of 250, 125, 62.5 and 31.25 µg/mL, using acetonitrile and water (1:1), to be used as the standard solutions.

In the preparation of a sample solution, about 20 mg of 2DG-PLGA as a sample was weighed, and 10 mL of Milli-Q water was added thereto. The resulting mixture was stirred using a vortex mixer for 30 seconds, and then subjected to an ultrasonic treatment for 30 minutes, to prepare a sample stock solution. To 1.0 mL of the thus prepared sample stock solution, acetonitrile was added to a total volume of 2 mL. The resulting liquid was centrifuged for 15 minutes at 20°C and at 10,000 × g. The supernatant obtained after centrifugation was filtered using a 0.2 µm membrane filter, and the resulting filtrate was used as the sample solution. The sample solution was used in the test with n = 3. The standard solutions and the sample solution were analyzed by high-speed liquid chromatography (HPLC) under the following conditions, and the concentration of 2DG in the sample solution was calculated from the calibration curve of the standard solutions. The content percentage of 2DG was calculated based on the weight of 2DG-PLGA and the concentration of 2DG.

### Conditions for HPLC

Apparatus: Shimadzu 10A series (manufactured by Shimadzu Corporation)
Detection: Shimadzu ELSD-LT
Gain: 8
Drift temperature: 40°C
Gas pressure: 350 kPa (measured value: 358 kPa)
Nebulizer gas: N2
Column: Shodex Asahipak NH2P-504D (150 mm × 4.6 mm)
Column temperature: 40°C
Mobile phase: A: water, B: acetonitrile
B. concentration: 75% → 69% (8 minutes) 69 → 75% (8 minutes → 16 minutes)
Flow rate: 1.0 mL/min
Column temperature: 40°C
Injection volume: 20 µL
Measurement time: 16 minutes

### (Results)

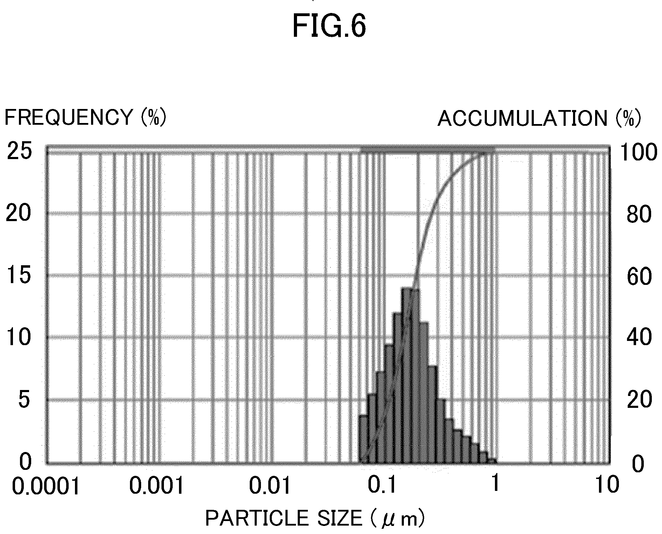

The content percentage of 2DG in 2DG-PLGA was 8.1 ± 0.4%. As shown in FIG. 6, the particle size distribution of 2DG-PLGA had a single peak, and the D₅₀ of 2DG-PLGA was 166 nm ± 5.4 nm. The span value of the particle size of 2DG-PLGA was 1.52 ± 0.15.

### (Example 3: Examination of Anticancer Effect of 2DG-PLGA, Using Nude Mice Subcutaneously Transplanted with Hepatoma Cell Line)

Nude mice subcutaneously transplanted with Huh-7 cells, prepared in in the same manner as in Example 1, were divided in the following six groups.
(g) Control group: 200 µL of PBS was administered into the jugular vein once a week.
(h) PLGA group: PLGA was administered into the jugular vein once a week.
(i) 2DG group: 100 mg/kg/day of the 100 mg/mL 2DG solution was administered into the jugular vein once a week.
(j) 2DG-PLGA group: 2DG-PLGA was administered into the jugular vein once a week.
(k) 2DG intraperitoneal injection group: 100 mg/kg/day of the 2DG solution was administered intraperitoneally every day.
(1) Sorafenib + PLGA group: 5 mg/kg/day of the sorafenib solution was administered orally every day, using a sonde, and PLGA was administered into the jugular vein once a week.
(m) Sorafenib + 2DG-PLGA group: 5 mg/kg/day of the sorafenib solution was administered orally every day, using a sonde, and 2DG-PLGA was administered into the jugular vein once a week.

The dose of 2DG administered in the groups (i) and (k), which is 100 mg/kg/day, substantially corresponds to a single dose of 2DG per mouse (calculated based on an average body weight of 25 g) of about 2.5 mg. Further, PLGA in the groups (h) and (l) and 2DG-PLGA in the groups (j) and (m) were each prepared as a 100 mg/mL suspension in PBS, every time before the administration, and 200 µL of the suspension was administered per mouse per administration. In view of the fact that the content percentage of 2DG in 2DG-PLGA is about 8%, the single dose of 2DG per mouse in the groups (j) and (m) substantially corresponds to about 1.6 mg, which is about 60% of that in the groups (i) and (k).

In each of the groups, the tumor volume and the body weight of the mice were measured in the same manner as in Example 1, and the average amount of meals taken per day was calculated. The blood glucose level of the mice in the 2DG-PLGA group was measured on day 14 after the start of administration, in the same manner as in Example 1. Twenty-one days after the start of administration, the tumors were observed, and the serological profiles and the liver weight were measured, in the same manner as in Example 1.

### (Results)

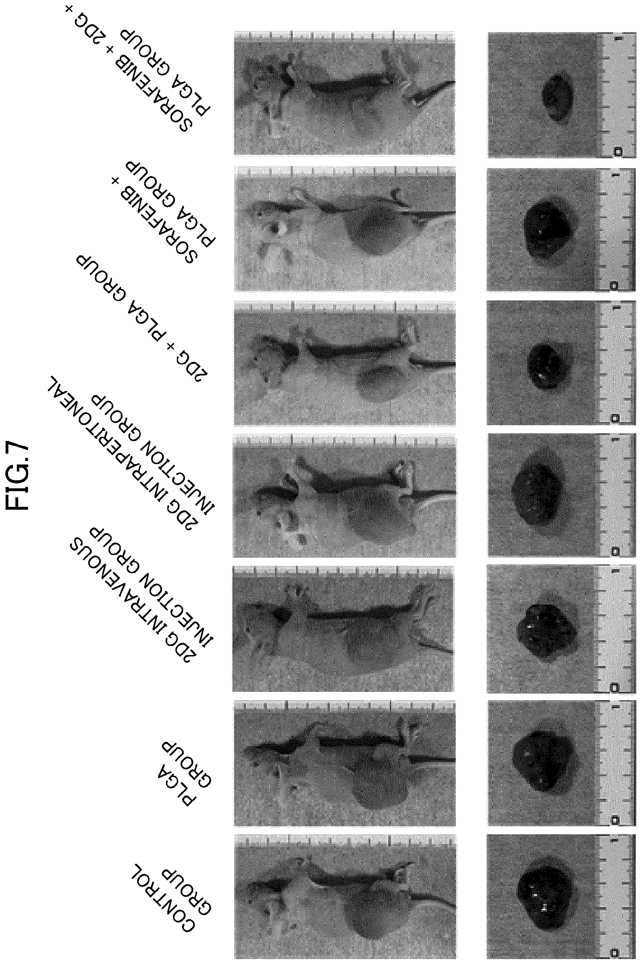

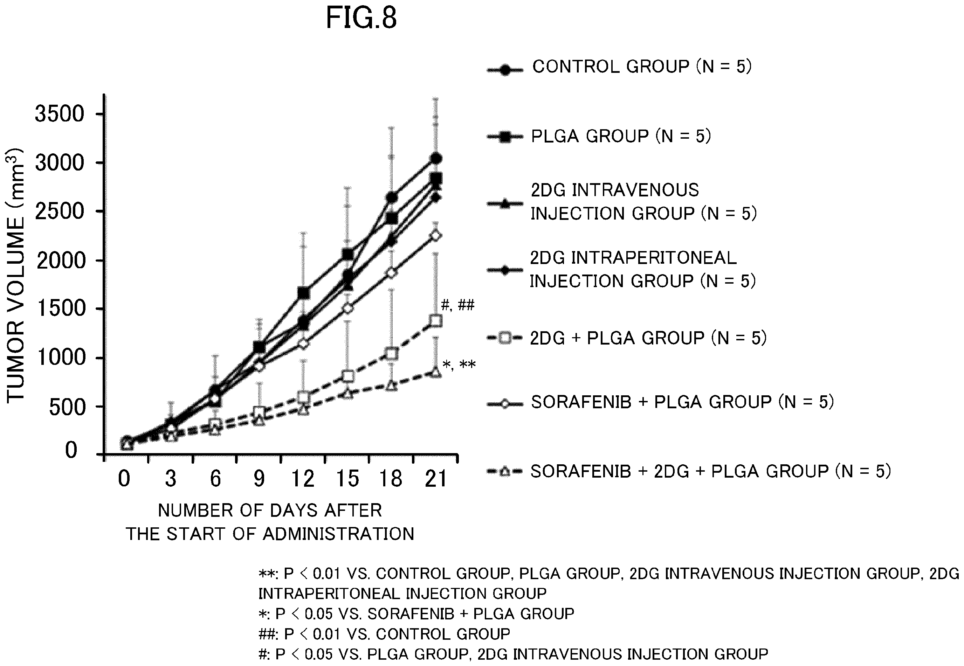

FIG. 7 shows the appearance of the tumors 21 days after the start of administration. 2DG-PLGA decreased the size of the tumors derived from the transplanted hepatoma cells. Further, 2DG-PLGA further decreased the tumor size when used in combination with sorafenib. FIG. 8 shows changes over time in the tumor volume. A significant decrease in the tumor volume was observed in the 2DG-PLGA group, as compared to the control group, the PLGA group and the 2DG intravenous injection group. A significant decrease in the tumor volume was also observed in the sorafenib + 2DG-PLGA group, in which sorafenib was used in combination.

In the tumor volume on day 21 after the start of administration, shown in FIG. 8, the sorafenib + PLGA group was significantly different from the control group with p < 0.01. In contrast, a further significant difference of p < 0.001 was observed between the sorafenib + 2DG-PLGA group and the control group. The above results have shown that the combined use of 2DG-PLGA with sorafenib provides a marked Anticancer effect.

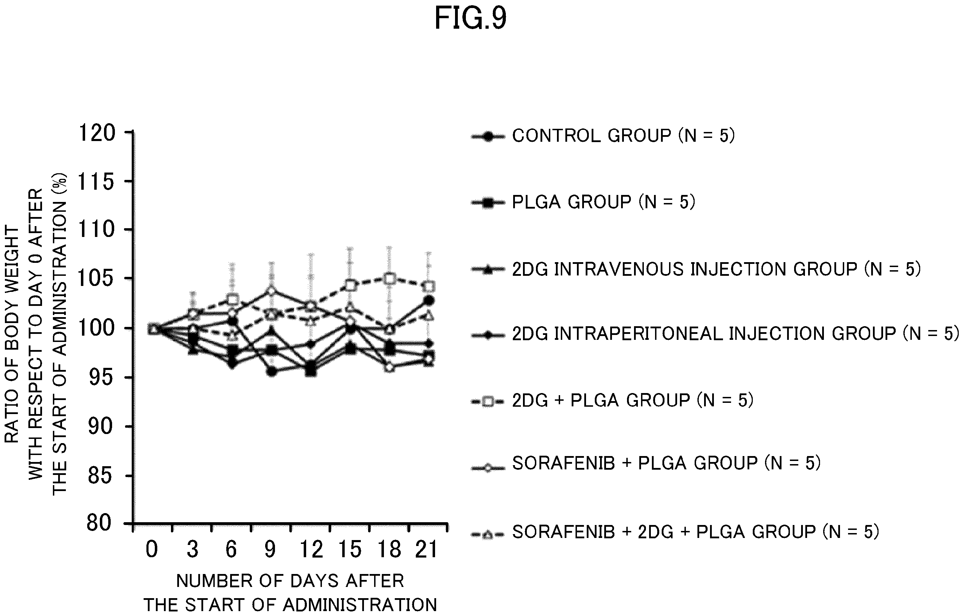

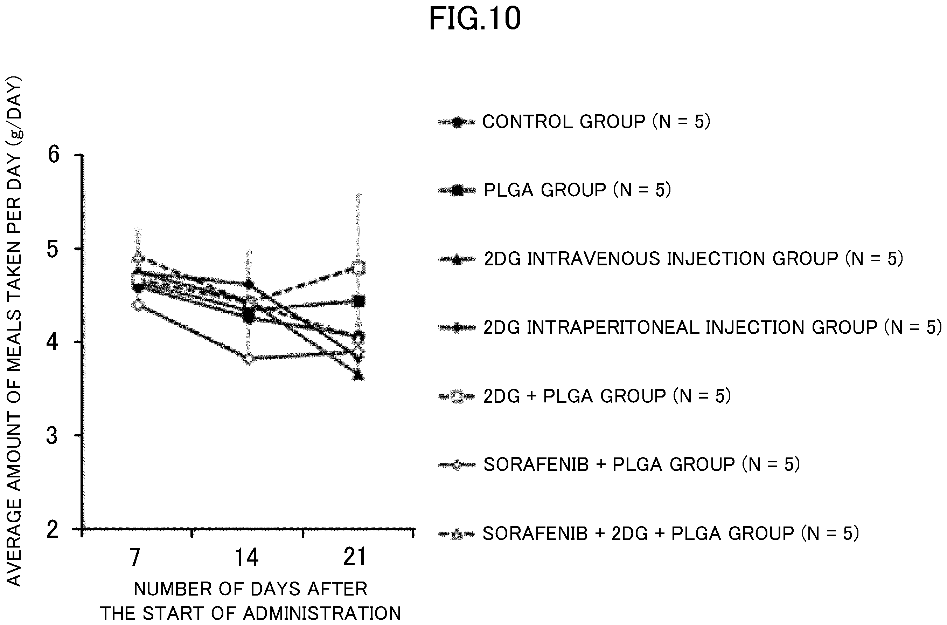

As shown in FIG. 9, no significant difference in the body weight of the mice was observed between all the groups, during the period of the experiment. Further, as shown in FIG. 10, no significant difference was observed between all the groups, also in the average amount of meals taken per day, at the time points of 7 days, 14 days and 21 days after the start of administration. The serological profiles and the liver weight at the time point of 21 days after the start of administration are shown in Table 2. No significant difference was observed in the serological profiles and the liver weight between all the groups, at the time point of 21 days after the start of administration.

**[Table 2]**

| | Contro l group | PLG A group | 2DG intravenou s injection group | 2DG intraperitonea l injection group | 2DG-PLG A group | Sorafeni b + PLGA group | Sorafenib + 2DG-PLG A group |
|---|---|---|---|---|---|---|---|
| ALT (mg/dL) | 37.0 ± 12.6 | 54.8 ± 39.5 | 49.0 ± 20.9 | 38.6 ± 23.5 | 45.8 ± 23.6 | 50.8 ± 38.8 | 47.4 ± 25.6 |
| Liver weight (g) | 1.25 ± 0.06 | 1.32 ± 0.05 | 1.10 ± 0.19 | 1.35 ± 0.19 | 1.35 ± 0.15 | 1.16 ± 0.21 | 1.19 ± 0.28 |
| Fasting blood glucose (mg/dL) | 73.6 ± 8.7 | 72.6 ± 86.3 | 73.8 ± 14.0 | 83.2 ± 38.2 | 72.8 ± 17.6 | 88.6 ± 10.1 | 80.6 ± 13.1 |
| Total cholestero 1 (mg/dL) | 124.2 ± 5.5 | 127.8 ± 20.4 | 115.4 ± 10.1 | 113.8 ± 25.3 | 124.6 ± 14.2 | 128.6 ± 13.8 | 117.2 ± 32.7 |
| Neutral fat (mg/dL) | 93.0 ± 17.8 | 93.6 ± 26.9 | 67.6 ± 28.5 | 85.2 ± 37.5 | 102.0 ± 33.4 | 85.2 ± 42.8 | 80.2 ± 71.1 |

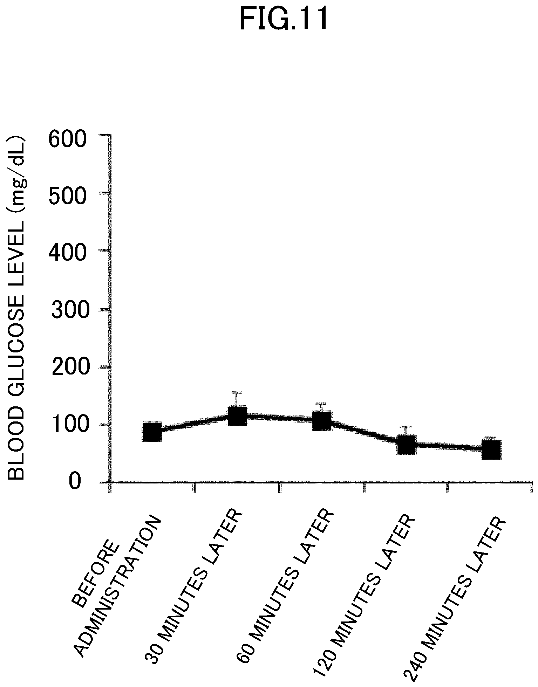

In the high dose 2DG group in Example 1, the disturbance of consciousness was observed along with a marked increase in the blood glucose level, after the administration of 2DG, on day 14 after the start of administration. In the present Example, however, neither an increase in the blood glucose level nor the disturbance of consciousness was observed in the 2DG-PLGA group, after the administration of 2DG-PLGA, on day 14 after the start of administration, as shown in FIG. 11. The above results have shown that the administration of 2DG-PLGA enables to provide a high anticancer effect, without causing side effects such as a decrease in body weight, liver damage and hyperglycemia.

### (Example 4: Examination of Anticancer Effect and Safety of Use of 2DG-PLGA in Combination with Anticancer Drug)

Mice subcutaneously transplanted with Huh-7 cells in the same manner as in Example 1 were divided into the following four groups, and the anticancer effect and the safety of the use of 2DG-PLGA in combination with an anticancer drug other than sorafenib were examined.

Doxorubicin + PLGA group: 4 mg/kg of a solution of doxorubicin (manufactured by Sigma-Aldrich Co. LLC) was administered orally and intraperitoneally every other day, and PLGA was administered into the jugular vein once a week.

Doxorubicin + 2DG-PLGA group: 4 mg/kg of the doxorubicin solution was administered orally and intraperitoneally every other day, and 2DG-PLGA was administered into the jugular vein once a week.

Cis-platin + PLGA group: 3 mg/kg of a solution of cis-platin (manufactured by Sigma-Aldrich Co. LLC) was administered intraperitoneally once a week, and PLGA was administered into the jugular vein once a week.

Cis-platin + 2DG-PLGA group: 3 mg/kg of the doxorubicin solution was administered intraperitoneally once a week, and 2DG-PLGA was administered into the jugular vein once a week.

In the administration of PLGA and 2DG-PLGA in the present Example, PLGA or 2DG-PLGA was prepared as a 100 mg/mL suspension in PBS, every time before the administration, and 200 µL of the suspension was administered per mouse per administration, in the same manner as in Example 3.

In each of the groups, the tumor volume, the body weight, the average amount of meals taken per day, the blood glucose level, the serological profiles and the liver weight were evaluated, and the tumors were observed, in the same manner as in Example 3. The anticancer effect and the safety in the respective groups in the present Example were compared with those in the control group and the like in the above described Example 3.

### (Results)

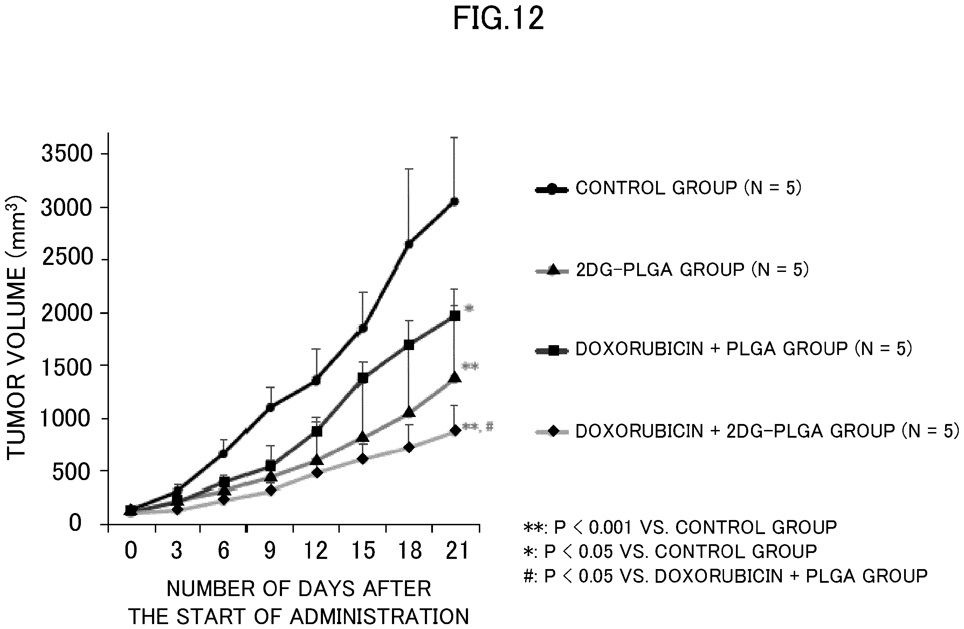

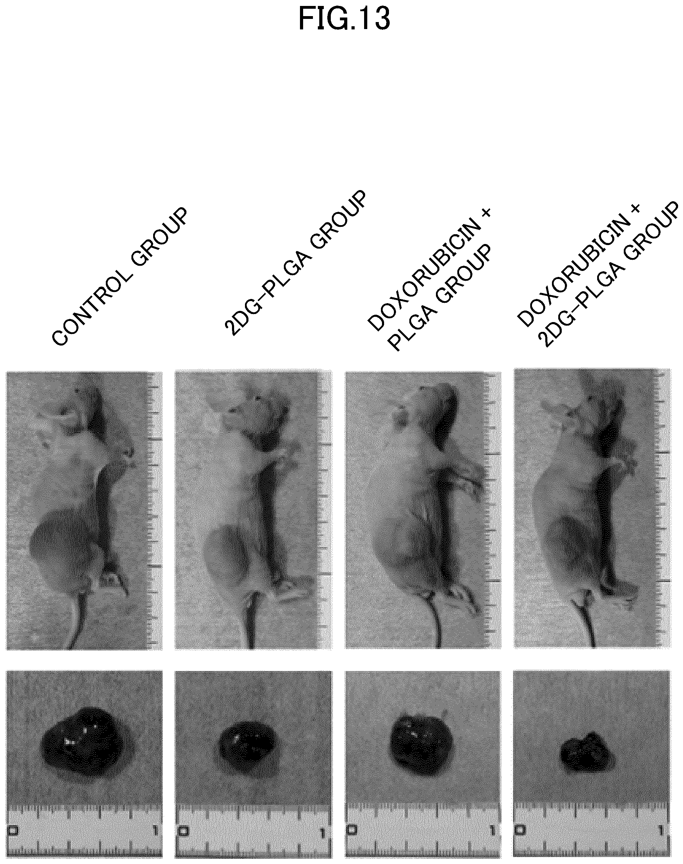

As shown in FIG. 12, a significant decrease in the tumor volume was observed in the 2DG-PLGA group and in the doxorubicin + 2DG-PLGA group, as compared to the control group. Further, a significant decrease in the tumor volume was observed in the doxorubicin + 2DG-PLGA group, as compared to the doxorubicin + PLGA group. As is evident from the appearance of the mice and the tumors 21 days after the start of administration, shown in FIG. 13, the administration of 2DG-PLGA enhances the anticancer effect of doxorubicin.

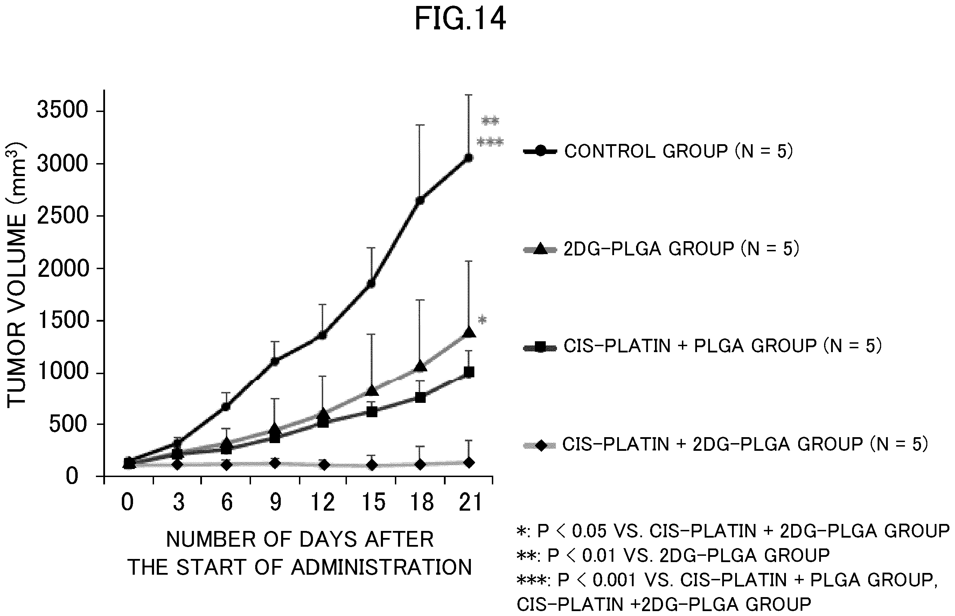

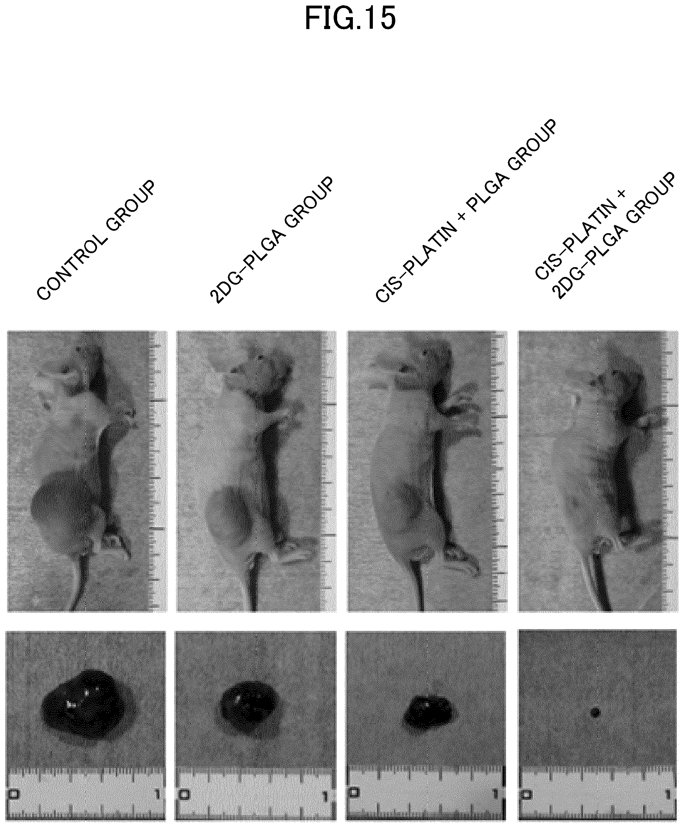

As shown in FIG. 14, a significant decrease in the tumor volume was observed in the 2DG-PLGA group and the cis-platin + 2DG-PLGA group, as compared to the control group. Further, the tumor volume in the cis-platin + 2DG-PLGA group was significantly different from the tumor volume in the 2DG-PLGA group. As is evident from the appearance of the mice and the tumors 21 days after the start of administration, shown in FIG. 15, the administration of 2DG-PLGA enhanced the anticancer effect of cis-platin, causing the tumors to nearly disappear.

In the body weight and the average amount of meals taken per day, no significant difference was observed between all the groups, including the groups (g) to (m) in the above described Example 3. The serological profiles and the liver weight at the time point of 21 days after the start of administration are shown in Table 3. No significant difference was observed between all the groups, in the serological profiles and the liver weight.

**[Table 3]**

| | Control group | PLGA group | Doxorubicin + PLGA group | Doxorubicin + 2DG-PLGA group | Cis-platin + PLGA group | Cis-platin + 2DG-PLGA group |
|---|---|---|---|---|---|---|
| ALT (mg/dL) | 37.0 ± 12.6 | 54.8 ± 39.5 | 36.0 ± 12.0 | 24.4 ± 11.5 | 27.6 ± 8.0 | 42.6 ± 30.2 |
| Liver weight (g) | 1.25 ± 0.06 | 1.32 ± 0.05 | 1.17 ± 0.25 | 1.45 ± 0.07 | 1.20 ± 0.15 | 1.30 ± 0.11 |
| Fasting blood glucose (mg/dL) | 73.6 ± 8.7 | 72.6 ± 86.3 | 87.8 ± 22.3 | 86.0 ± 8.5 | 82.2 ± 10.7 | 86.2 ± 5.0 |
| Total cholesterol (mg/dL) | 124.2 + 55 | 127.8 ± 20.4 | 113.0 ± 15.2 | 106.2 ± 6.2 | 117.0 ± 9.9 | 106.0 ± 19.5 |
| Neutral fat (mg/dL) | 93.0 ± 17.8 | 93.6 ± 26.9 | 86.6 ± 44.4 | 109.8 ± 41.7 | 96.6 ± 20.3 | 95.8 ± 29.3 |

### (Example 5: Examination of Anticancer Effect of 2DG-PLGA, Using Nude Mice Subcutaneously Transplanted with Renal Cancer Cell Line)

The renal cancer cell line OS-RC-2 (obtained from RIKEN) was cultured at 37°C using a 5% CO₂ incubator. As the medium for culturing OS-RC-2 cells, RPMI containing 10% fetal bovine serum was used.

Six-week-old nude mice (male, BALBc-nu/nu mice) were subcutaneously transplanted with 5 × 10⁶ OS-RC-2 cells. The measurement of the tumor size was carried out using a pair of vernier calipers, and at the time point when the tumors in the mice had reached a volume of from 100 to 150 mm³, the mice were divided into the following three groups.

Control group: 200 µL of PBS was administered into the jugular vein once a week.

2DG group: 100 mg/kg/day of a 100 mg/mL 2DG solution was administered intraperitoneally every day.

2DG-PLGA group: 2DG-PLGA was administered into the jugular vein once a week.

The administration of 2DG-PLGA in the 2DG-PLGA group was carried out in the same manner as in Example 3. In each of the groups, the tumor volume and the body weight of the mice were measured in the same manner as in Example 1. Fourteen days after the start of administration, the mice were euthanized and the tumors were observed, in the same manner as in Example 1.

### (Results)

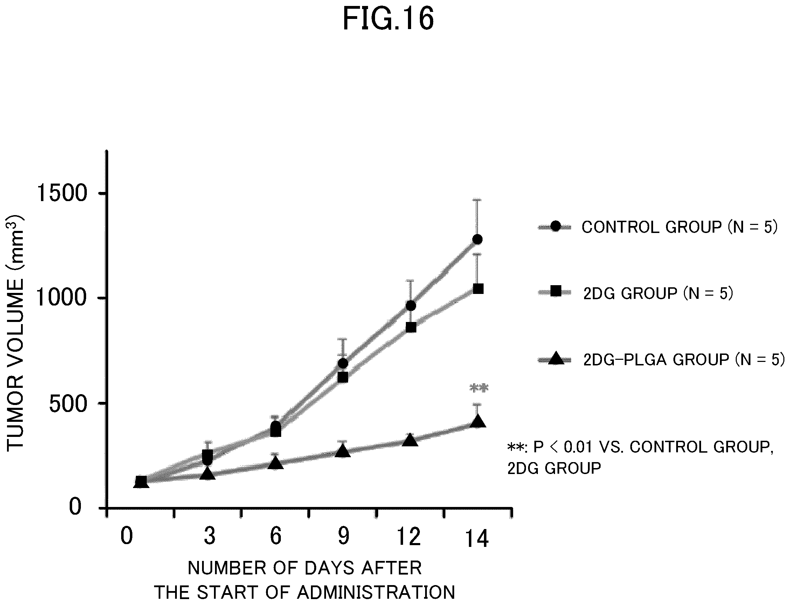

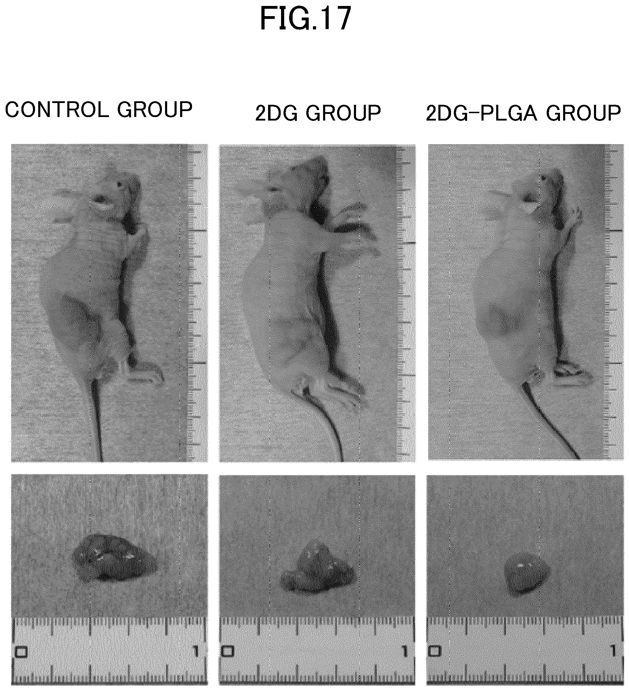

FIG. 16 shows changes over time in the tumor volume. A significant decrease in the tumor volume was observed in the 2DG-PLGA group, as compared to the control group and the 2DG group. FIG. 17 shows the appearance of the mice and the tumors 14 days after the start of administration. The tumor size in the 2DG-PLGA group was clearly smaller as compared to the tumor size in the 2DG group. 2DG-PLGA showed an anticancer effect also in the nude mice subcutaneously transplanted with the renal cancer cell line OS-RC-2.

### (Example 6: Examination of Anticancer Effect of 2DG-PLGA, Using Nude Mice Subcutaneously Transplanted with Colorectal Cancer Cell Line)

The colorectal cancer cell line HT-29 was cultured at 37°C using a 5% CO₂ incubator. As the medium for culturing HT-29 cells, McCoy's 5A containing 10% fetal bovine serum was used. The anticancer effect of 2DG-PLGA was evaluated in the same manner as in the above described Example 5, except that the mice were transplanted with HT-29 cells instead of OS-RC-2 cells, and that the mice were euthanized 21 days after the start of administration, instead of 14 days thereafter.

### (Results)

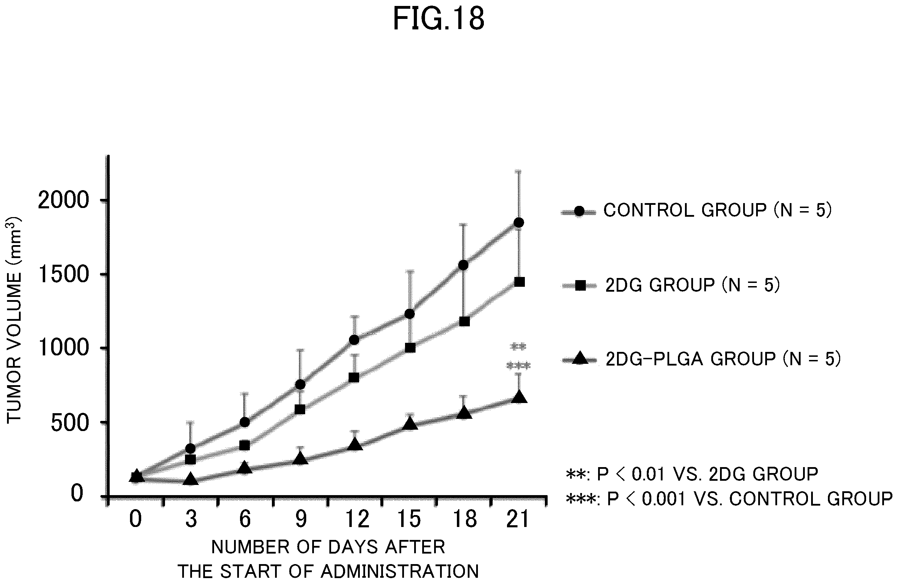

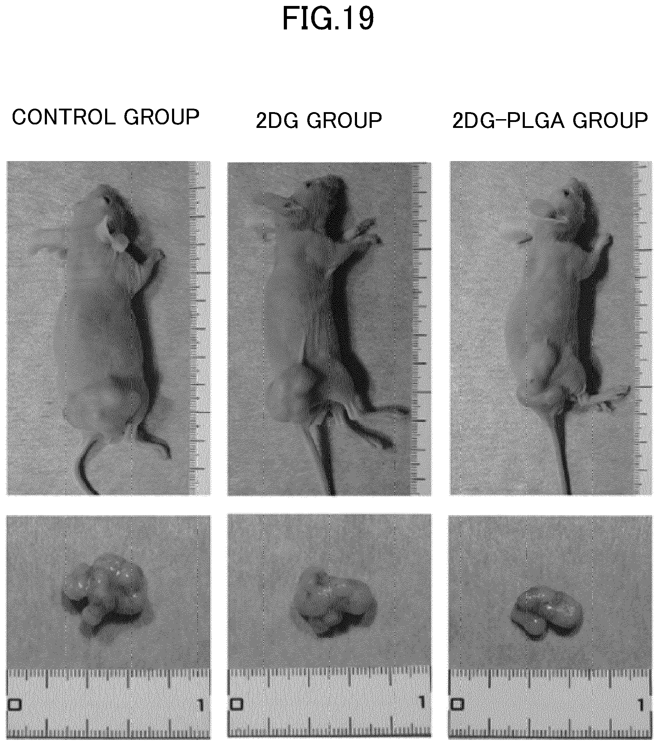

FIG. 18 shows changes over time in the tumor volume. A significant decrease in the tumor volume was observed in the 2DG-PLGA group, as compared to the control group and the 2DG group. FIG. 19 shows the appearance of the mice and the tumors 21 days after the start of administration. The tumor size in the 2DG-PLGA group was clearly smaller as compared to the tumor size in the 2DG group. 2DG-PLGA showed an anticancer effect also in the nude mice subcutaneously transplanted with the colorectal cancer cell line HT-29.

### (Example 7: Examination of Anticancer Effect of 2DG-PLGA, Using Nude Mice Subcutaneously Transplanted with Pancreatic Cancer Cell Line)

The pancreatic cancer cell line Bx-PC-3 was cultured at 37°C using a 5% CO₂ incubator. As the medium for culturing Bx-PC-3 cells, RPMI containing 10% fetal bovine serum was used. The anticancer effect of 2DG-PLGA was evaluated in the same manner as in the above described Example 6.

### (Results)

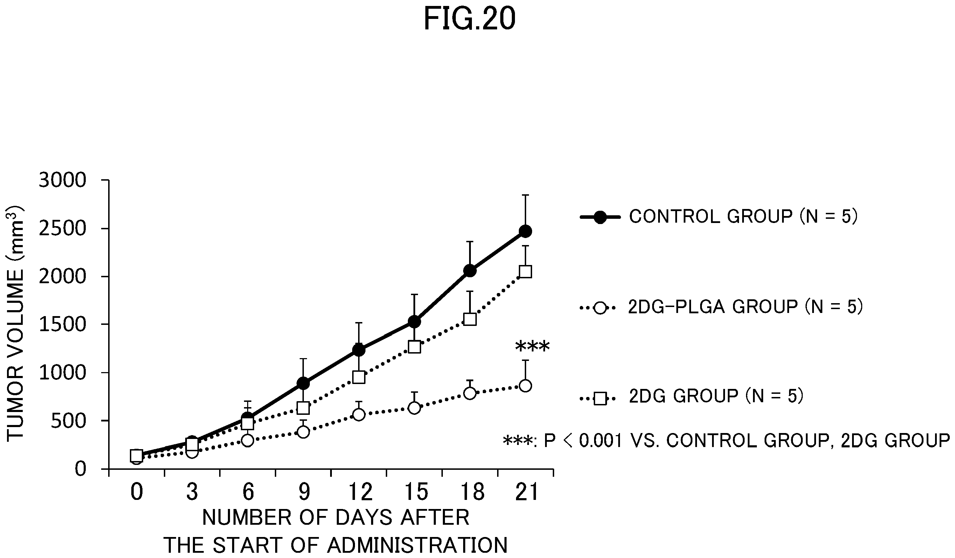

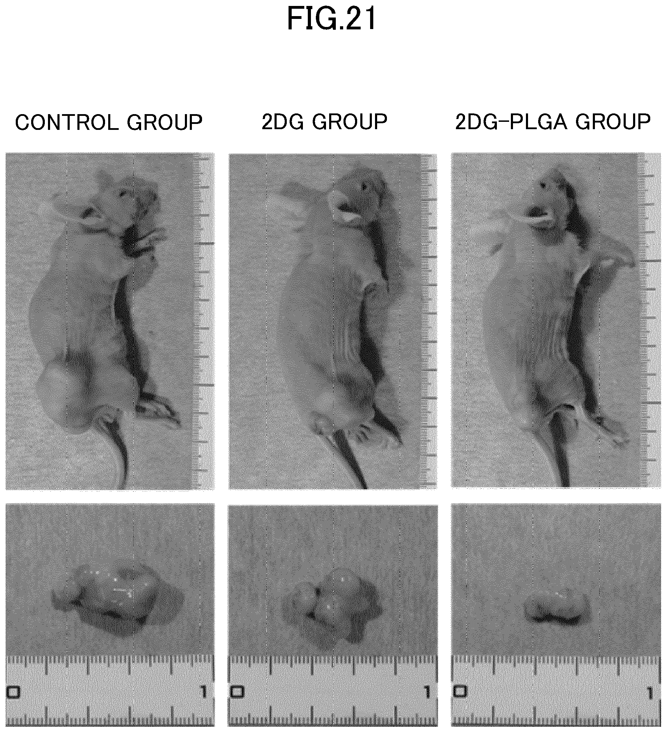

FIG. 20 shows changes over time in the tumor volume. A significant decrease in the tumor volume was observed in the 2DG-PLGA group, as compared to the control group and the 2DG group. FIG. 21 shows the appearance of the mice and the tumors 21 days after the start of administration. The tumor size in the 2DG-PLGA group was clearly smaller as compared to the tumor size in the 2DG group. 2DG-PLGA showed an anticancer effect also in the nude mice subcutaneously transplanted with the pancreatic cancer cell line Bx-PC-3.

### (Example 8: Analysis of Biodistribution of ICG-PLGA in Tumors and in Mice, Using Nude Mice Subcutaneously Transplanted with Hepatoma cell line)

PLGA particles encapsulating indocyanine green (hereinafter referred to as "ICG"; manufactured by Daiichi Sankyo Company Limited) were prepared, using ULREA SS-11 (manufactured by M Technique Co., Ltd.). An aqueous dispersion of ICG-PLGA, which was obtained from: an aqueous solution containing 0.5% PVA prepared in the same manner as in Example 2 (Liquid A); and a solution containing PLGA, ICG, acetone and ethanol in a weight ratio of 0.65:0.1:66:33 (Liquid B); was freeze dried, to obtain 0.61 g of ICG-PLGA.

The resulting ICG-PLGA was analyzed by high-speed liquid chromatography (HPLC), and the content percentage (content) of ICG in a sample solution was calculated from the calibration curve of standard solutions. The content of ICG in the ICG-PLGA was 6.2 ± 0.3%.

The particle size distribution of ICG-PLGA was measured using NIKKISO Nanotrac Wave-EX1 50 (manufactured by Nikkiso Co., Ltd.). The particle size distribution of ICG-PLGA had a single peak, and the D₅₀ of ICG-PLGA was 187 nm. The span value of the particle size of ICG-PLGA was 1.5 nm.

Eight-week-old nude mice (male, BALBc-nu/nu mice) were subcutaneously transplanted with 1 × 10⁷ Huh-7 cells. The measurement of the tumor size was carried out using a pair of vernier calipers. After confirming that the maximum diameter of the tumors in the mice had reached a size of about 15 mm, ICG-PLGA was injected to the jugular vein of each mouse (800 mg/kg). Changes over time in the biodistribution of ICG-PLGA in the tumors and in the mice were analyzed using an in vivo imaging system (IVIS), at the time points of 1.5 hours, 6 hours, 24 hours, 2 days, 3 days and 7 days after the injection, under isoflurane inhalation anesthesia. IVIS Spectrum (manufactured by Xenogen Corporation) was used as the IVIS.

### (Results)

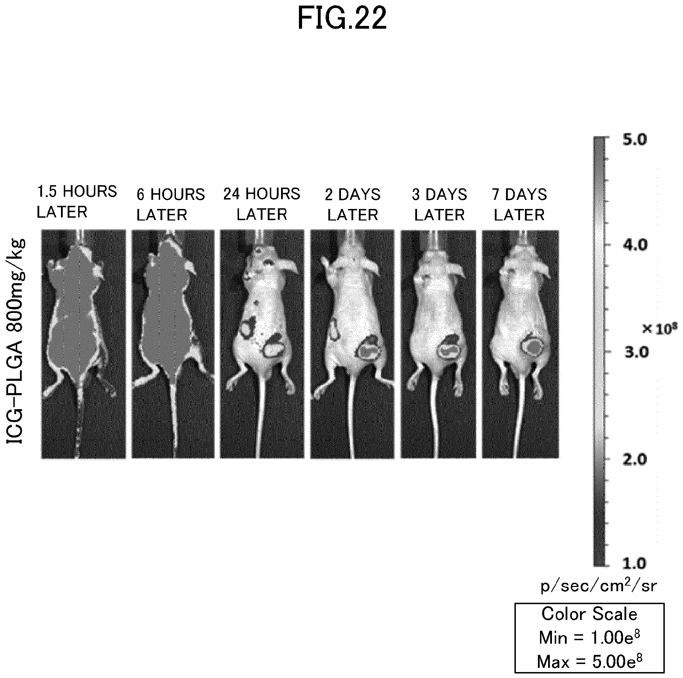

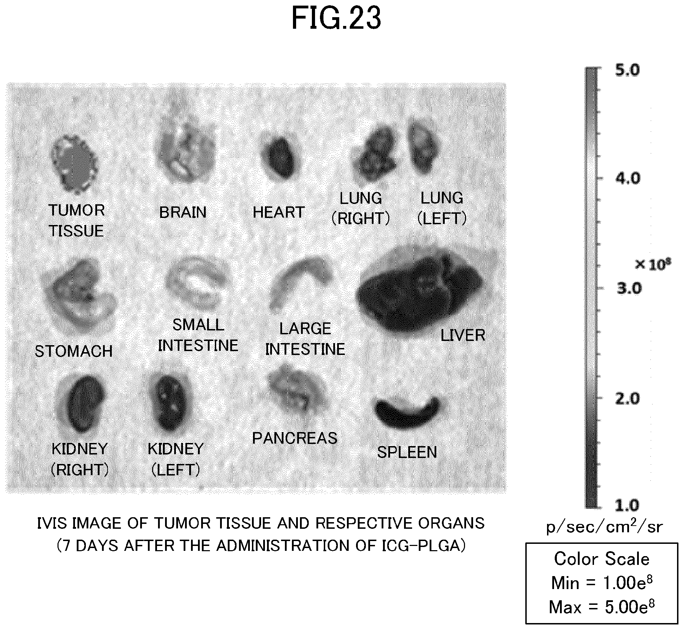

FIG. 22 and FIG. 23 show the analysis results by IVIS. As shown in FIG. 22, although the distribution of ICG was observed all over the body of the mouse, by IVIS, from immediately after the administration of ICG-PLGA, ICG-PLGA accumulated in the tumors from immediately after the administration. Seven days after the administration, the accumulation of ICG was still observed in the tumor portion, but the accumulation of ICG was not observed in any other biological tissue. Further, the tumor tissue and respective organs were collected 7 days after the administration of ICG-PLGA, and images were captured by IVIS. As a result, an intense accumulation was observed only in the tumor tissue, and no obvious accumulation was observed in the respective organs other than the tumor tissue, as shown in FIG. 23.

### (Example 9: Examination of Anticancer Effect of 2DG-PLGA Varying in Particle Size)

Six-week-old nude mice (male, BALBc-nu/nu mice) were subcutaneously transplanted with 1 × 10⁷ Huh-7 cells. The measurement of the tumor size was carried out using a pair of vernier calipers, and at the time point when the tumors in the mice had reached a volume of from about 100 to 150 mm³, the mice were divided into the following three groups.

Control group: 200 µL of PBS was administered into the jugular vein once a week.

2DG-PLGA (D₅₀ = 49 nm) group: 2DG-PLGA having a D₅₀ of 49 nm was administered into the jugular vein once a week (800 mg/kg). The content percentage of 2DG in this 2DG-PLGA was 8.5 ± 0.5%, and the span value of the 2DG-PLGA was 0.95.

2DG-PLGA (D₅₀ = 153 nm) group: 2DG-PLGA having a D₅₀ of 153 nm was administered into the jugular vein once a week (800 mg/kg). The content percentage of 2DG in this 2DG-PLGA was 7.2 ± 0.2%, and the span value of the 2DG-PLGA was 1.3.

2DG-PLGA (D₅₀ = 313 nm) group: 2DG-PLGA having a D₅₀ of 313 nm was administered into the jugular vein once a week (800 mg/kg). The content percentage of 2DG in this 2DG-PLGA was 6.6 ± 0.2%, and the span value of the 2DG-PLGA was 1.6.

In the administration of the respective 2DG-PLGAs in the present Example, each 2DG-PLGA was prepared as a 100 mg/mL suspension in PBS, every time before the administration, and the suspension was administered such that 800 mg/kg of each 2DG-PLGA was administered per mouse per administration. In each of the above described groups, the tumor volume of the mice was measured every 3 days from the start of administration, and changes over time in the tumor volume were evaluated. Twenty-one days after the start of administration, the mice were euthanized, and the tumors were observed.

### (Results)

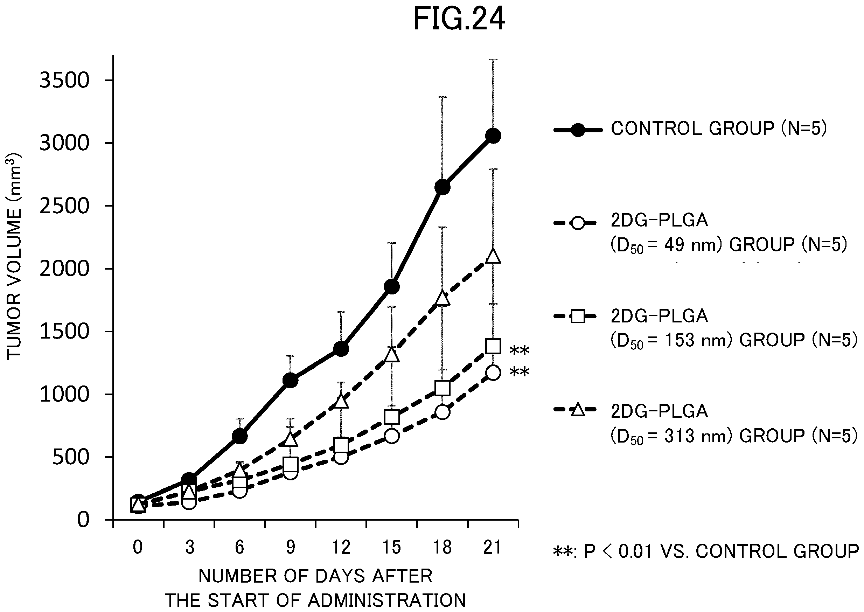

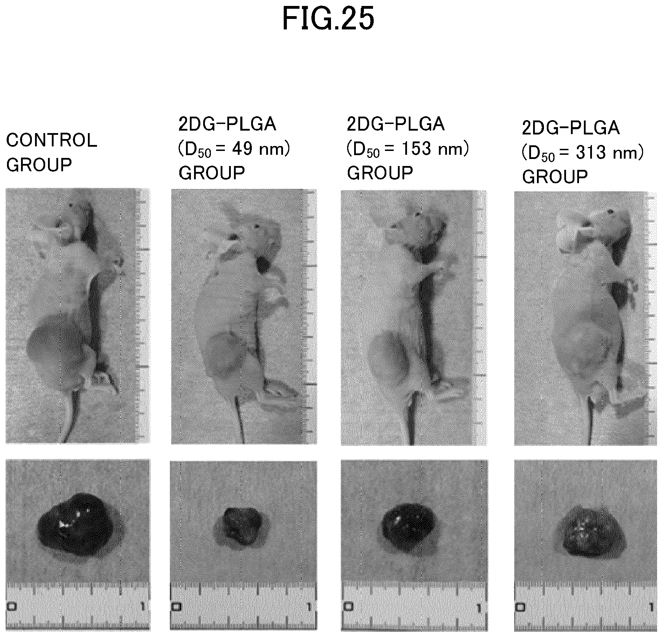

FIG. 24 shows changes over time in the tumor volume. A significant decrease in the tumor volume was observed in the 2DG-PLGA (D₅₀ = 49 nm) group and the 2DG-PLGA (D₅₀ = 153 nm) group, as compared to the control group and the 2DG-PLGA (D₅₀ = 313 nm) group. FIG. 25 shows the appearance of the mice and the tumors 21 days after the start of administration. In the 2DG-PLGA (D₅₀ = 49 nm) group and the 2DG-PLGA (D₅₀ = 153 nm) group, a significant decrease was observed in the size of the tumors derived from hepatoma cells transplanted in the same manner, as compared to the control group. In the 2DG-PLGA (D₅₀ = 313 nm) group, although a decrease in the tumor volume was observed as compared the control group, the extent of the decrease was not as large as that in the 2DG-PLGA (D₅₀ = 50 nm) group and in the 2DG-PLGA (D₅₀ = 153 nm) group.

### (Example 10: Examination of Anticancer Effect of 2DG-PLGA, Using Hepatocarcinogenic Mice Without Immunodeficiency)

In order to evaluate the anticancer immunity in the anticancer effect of 2DG-PLGA, an experiment was carried out using hepatocarcinogenic mice without immunodeficiency.

STAM (trademark) mice (produced by SMC laboratories, Inc.) were obtained as the hepatocarcinogenic mice. These STAM mice were produced by subcutaneously injecting streptozotocin to two days-old, male C57BL6/N mice, weaning the mice at 4 weeks after the birth, and start feeding a high fat diet.

The twelve-week-old hepatocarcinogenic (male) mice were divided into the following four groups.
(1) Control group: 200 µL of PBS was administered into the jugular vein once a week.
(2) 2DG group: 100 mg/kg/ day of a 100 mg/mL 2DG solution was administered intraperitoneally every day.
(3) Low dose 2DG-PLGA group: 2DG-PLGA was administered into the jugular vein once a week.
(4) High dose 2DG-PLGA group: 2DG-PLGA was administered into the jugular vein once a week, in a dose 10 times the dose used in the group (3).

The concentration of 2DG administered in the group (2), which is 100 mg/kg/day, substantially corresponds to a single dose of 2DG per mouse (calculated based on an average body weight of 27 g) of about 2.7 mg. 2DG-PLGA in the group (3) was prepared as a 10 mg/mL suspension in PBS, every time before the administration, and 200 µL of the suspension was administered per mouse per administration. 2DG-PLGA in the group (4) was prepared as a 100 mg/mL suspension in PBS, every time before the administration, and 200 µL of the suspension was administered per mouse per administration.

In view of the fact that the filling rate of 2DG in 2DG-PLGA is about 8%, the single dose of 2DG per mouse in the group (3) substantially corresponds to about 0.16 mg, which is about 6% of that in the group (2). The single dose of 2DG per mouse in the group (4) substantially corresponds to about 1.6 mg, which is about 60% of that in the group (2).

In each of the groups (1) to (4), the entire liver was observed in each mouse on day 21 from the start of administration, and the maximum tumor diameter, the number of tumors and the total tumor volume in the liver were measured (n = 5). Further, the immunostaining of the liver tumor tissue was carried out using a rabbit monoclonal CD3 antibody (manufactured by GeneTex, Inc.).

### (Results)

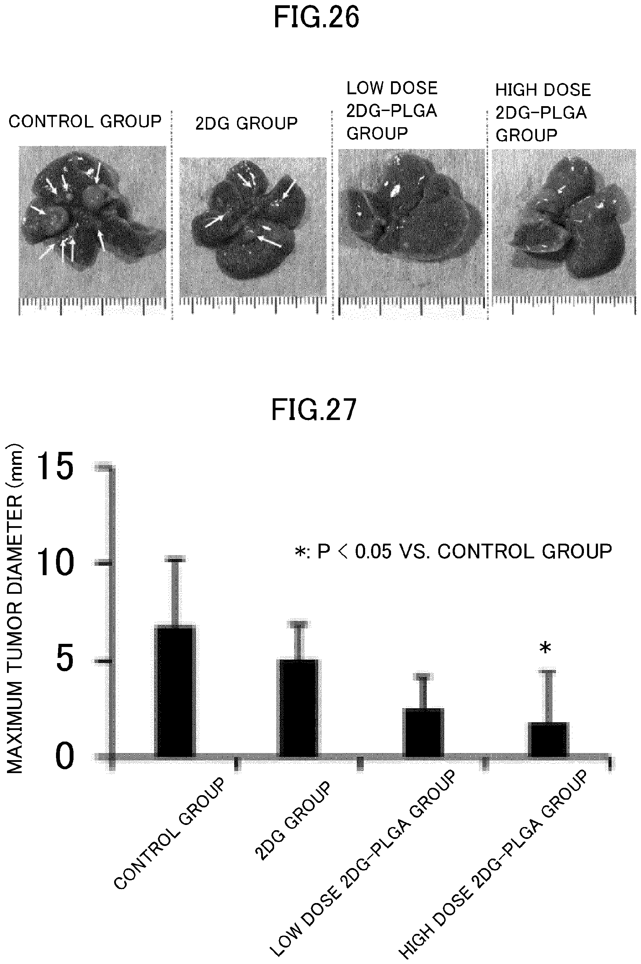

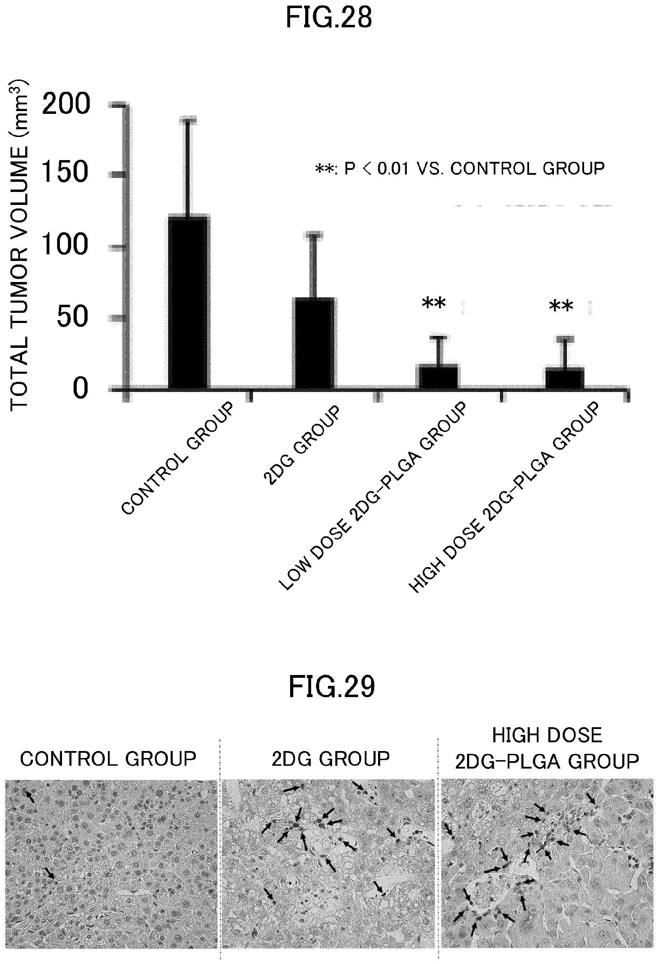

FIG. 26 shows the appearance of the tumors 21 days after the start of administration. 2DG-PLGA inhibited an increase in the number of the tumors not only in the high dose group but also in the low dose group. As shown in FIG. 27, 2DG-PLGA decreased the maximum tumor diameter in both the high dose group and the low dose group. In particular, a significant decrease in the maximum tumor diameter was observed in the high dose group, as compared to the control group. Further, as shown in FIG. 28, 2DG-PLGA significantly decreased the total tumor volume not only in the high dose group but also in the low dose group, as compared to the control group.

FIG. 29 shows the results of the immunostaining. In FIG. 29, CD3 positive T cells stained by immunostaining are indicated with arrows. An increased infiltration of T cells into the interior of tumors was observed, in the 2DG group and in the high dose 2DG-PLGA group. The hepatocarcinogenic model mice used in the present Example are mice without immunodeficiency including T cell immunodeficiency. In the present Example, 2DG-PLGA showed the same anticancer effect as the effect shown at the high dose in Example 1, even at a low dose corresponding to 1/10 of the dose of 2DG-PLGA administered to the immunodeficient nude mice subcutaneously transplanted with the hepatoma cell line in Example 1. The reason for this is thought to be because, as shown in FIG. 29, the effect of promoting T cell-based anticancer activity, which effect was not observed in the immunodeficient nude mice subcutaneously transplanted with the hepatoma cell line, worked synergistically with the mechanism of the anticancer effect.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2017-125770, filed on June 28, 2017, and Japanese Patent Application No. 2017-195178, filed on October 5, 2017, of which the entirety of the disclosures is incorporated by reference herein.

### Industrial Applicability

The present disclosure is suitable for use as a pharmaceutical composition, in particular, for use as an anticancer drug.

## Claims

1. A pharmaceutical composition comprising biocompatible particles containing a sugar derivative,
wherein the biocompatible particles contain a lactic acid-glycolic acid copolymer.

2. The pharmaceutical composition according to claim 1, wherein the pharmaceutical composition is used in combination with an anticancer drug.

3. A pharmaceutical composition comprising an anticancer drug, wherein the pharmaceutical composition is used in combination with the pharmaceutical composition according to claim 1.

4. A pharmaceutical composition comprising:
biocompatible particles containing a sugar derivative; and
an anticancer drug;
wherein the biocompatible particles contain a lactic acid-glycolic acid copolymer.

5. The pharmaceutical composition according to any one of claims 2 to 4, wherein the anticancer drug is sorafenib, doxorubicin or cis-platin.

6. The pharmaceutical composition according to any one of claims 1 to 5, wherein the sugar derivative is 2-deoxy-D-glucose.

7. The pharmaceutical composition according to any one of claims 1 to 6, wherein the biocompatible particles have an average particle size of from 50 to 170 nm.

8. The pharmaceutical composition according to any one of claims 1 to 7, wherein the pharmaceutical composition is used for treating hepatocellular carcinoma, renal cancer, colorectal cancer or pancreatic cancer.

9. An anticancer immune activator comprising biocompatible particles containing a sugar derivative, wherein the biocompatible particles contain a lactic acid-glycolic acid copolymer.
